(19) Europäisches Patentamt
European Patent Office
Office européen des brevets

(11) Veröffentlichungsnummer: **0 249 978**
**A2**

(12) # EUROPÄISCHE PATENTANMELDUNG

(21) Anmeldenummer: **87108761.5**

(51) Int. Cl.⁴: **C07D 233/64**

(22) Anmeldetag: **19.06.87**

(30) Priorität: **18.06.86 DE 3620430**

(43) Veröffentlichungstag der Anmeldung:
**23.12.87 Patentblatt 87/52**

(84) Benannte Vertragsstaaten:
**BE CH DE FR GB IT LI NL**

(71) Anmelder: **BASF Aktiengesellschaft**
**Carl-Bosch-Strasse 38**
**D-6700 Ludwigshafen(DE)**

(72) Erfinder: **Kempe, Uwe, Dr.**
**Danziger Strasse 9**
**D-6701 Dannstadt-Schauernheim(DE)**
Erfinder: **Dockner, Toni, Dr.**
**Grossgasse 6**
**D-6701 Meckenheim(DE)**
Erfinder: **Karn, Helmut**
**Sternstrasse 120**
**D-6700 Ludwigshafen(DE)**
Erfinder: **Bluemel, Thomas, Dr.**
**An der Bleiche 12 a**
**D-6701 Erpolzheim(DE)**
Erfinder: **Leyrer, Reinhold J., Dr.**
**Menzelstrasse 4**
**D-6700 Ludwigshafen(DE)**
Erfinder: **Loerzer, Thomas, Dr.**
**Carl-Bosch-Ring 2**
**D-6710 Frankenthal(DE)**

(54) **Verfahren zur Herstellung von Hexaarylbisimidazolen.**

(57) Herstellung von Hexaarylbisimidazolen I

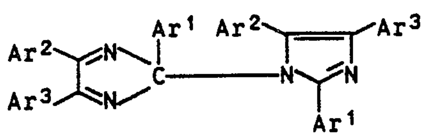

I,

mit Ar¹ bis Ar³ = Arylreste, wobei Ar² und Ar³ zu einem anellierten Ringsystem verknüpft sein können, durch Oxidation der entsprechenden Triarylimidazole II

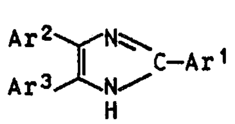

II

in einem wäßrig-organischen Zweiphasensystem in Gegenwart wirksamer Mengen eines Oniumsalzes III.

EP 0 249 978 A2

## Verfahren zur Herstellung von Hexaarylbisimidazolen

Die vorliegende Erfindung betrifft ein verbessertes Verfahren zur Herstellung von Hexaarylbisimidazolen der Formel I

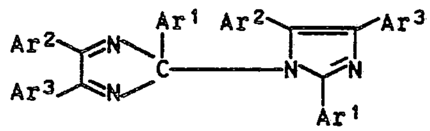

I,

in der $Ar^1$ bis $Ar^3$ Arylreste bezeichnen, wobei $Ar^2$ und $Ar^3$ auch miteinander zu einem anellierten Ringsystem verknüpft sein können, durch Oxidation der entsprechenden Triarylimidazole II

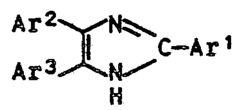

II

in einem wäßrig-organischen Zweiphasensystem.

Es ist allgemein bekannt, z.B. aus Bull. Soc. Chem. Jap. 43, 428-36 (1970), daß Hexaarylbisimidazole durch Oxidation der entsprechenden Triarylimidazole synthetisiert werden können. Die Oxidation erfolgt entweder in einem homogenen Lösungsmittel (z.B. Benzol) oder Lösungsmittelgemisch (z.B. Wasser/Ethanol) oder in einem wäßrig-organischen Zweiphasensystem, wobei als organische Phase Benzol verwendet wird. Die zweiphasige Umsetzung ist wegen ihrer größeren Anwendungsbreite und zur Herstellung größerer Mengen vorteilhafter wie aus J. Org. Chem. 36 , S. 2262 bis 2267 (1971) hervorgeht. Nachteilig an diesem Verfahren sind jedoch die langen Reaktionszeiten von 16 h.

Als Oxidationsmittel im heterogenen System werden wäßrige, alkalische Kaliumhexacyanoferrat-III-Lösungen verwendet. Oxidationsmittel in homogener Phase sind darüber hinaus Hypohalogenite oder Bleidioxid.

Der Erfindung lag die Aufgabe zugrunde, ein effektives Verfahren zur Herstellung von Hexaarylbisimidazolen zu finden, das es gestattet, die Umsetzung im technischen Maßstab bei möglichst kurzen Reaktionszeiten und hohen Ausbeuten durchzuführen.

Demgemäß wurde ein Verfahren zur Herstellung von Hexaarylbisimidazolen der Formel I

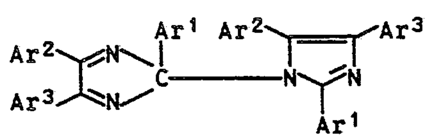

I,

in der $Ar^1$ bis $Ar^3$ Arylreste bezeichnen, wobei $Ar^2$ und $Ar^3$ auch miteinander zu einem anellierten Ringsystem verknüpft sein können, durch Oxidation der entsprechenden Triarylimidazole II

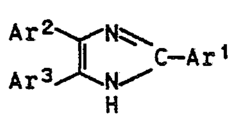

II

in einem wäßrig-organischen Zweiphasensystem gefunden, das dadurch gekennzeichnet ist, daß man die Oxidation in Gegenwart wirksamer Mengen eines Oniumsalzes III vornimmt.

Als Oniumsalze III eignen sich prinzipiell alle Verbindungen dieses Typs, darunter besonders quartäre Ammonium-, Phosphonium-, Arsonium-und ternäre Sulfoniumsalze. Die bevorzugten Oniumsalze lassen sich durch die allgemeinen Formeln IIIa und IIIb wiedergeben

$R_4M^{\oplus} X^{\ominus}$ IIIa

$R_3S^{\oplus} X^{\ominus}$ IIIb,

in denen die Reste R gleiche oder verschiedene Kohlenwasserstoffreste, z.B. Alkyl-, Cycloalkyl-, Aryl-oder Aralkylreste bedeuten, M für Phosphor, Arsen oder Stickstoff steht und X⊖ das Anionäquivalent einer Mineralsäure darstellt.

Allgemein können die Kohlenwasserstoffreste in den Verbindungen IIIa oder IIIb verzweigte oder vorzugsweise unverzweigte $C_1$-$C_{20}$-Alkylgruppen, die $C_5$-oder $C_6$-Cycloalkylgruppen, Arylgruppen oder Aralkylgruppen mit 6 bzw. 7 bis 20 Kohlenstoffatomen z.B. die Phenyl-, die p-Tolyl-oder die Benzylgruppe bedeuten. Zwei der Alkylreste R können auch miteinander verbunden sein, z.B. unter Ausbildung eines 5- oder 6-gliedrigen Ringes, z.B. eines Piperidinringes. Vorzugsweise ist die Summe der C-Atome in den Resten R nicht größer als 25.

Als Anionen X⊖ kommen insbesondere die Anionen organischer oder vorzugsweise anorganischer, einbasiger Säuren in Betracht. Beispielsweise seien F⊖, Cl⊖, Br⊖, J⊖, $NO_3$⊖, $HSO_4$⊖, $ClO_4$⊖, $HCO_3$⊖, $CH_3CO$⊖$_2$ oder $C_6H_5CO_2$⊖ aufgeführt. Eine Zusammenstellung weiterer Anionen findet man in E.V. Dehmlow und S.S. Dehmlow, Phase Transfer Catalysis, 2. Aufl., S. 14-16, 1983.

Aufgrund der generellen Eignung der Oniumsalze richtet sich deren Wahl hauptsächlich nach deren Verfügbarkeit und nach deren Preis. Praktisch wird man daher vor allem die Ammoniumsalze, insbesondere die Ammoniumbromide und -chloride einsetzen, wobei das handelsübliche und leicht herzustellende Tetrabutylammoniumbromid an erster Stelle zu nennen ist. Daneben sind diejenigen Ammoniumsalze hervorzuheben, in denen drei der Reste R niedere Alkylreste wie die Methyl-, Ethyl-, Propyl-oder Butylgruppe und der vierte den Benzylrest oder einen unverzweigten $C_6$-$C_{19}$-Alkylrest bedeuten.

Unter den Phosphonium-und Arsoniumsalzen sind diejenigen am besten zugänglich, die sich vom Triphenylphosphin bzw. -arsin ableiten und deren vierter Substituent durch Quaternierung z.B. mit einem $C_1$-$C_7$-Alkylbromid in das Molekül eingeführt wird. Weiterhin seien Tetraphenylphosphonium-und Tetraphenylarsoniumhalogenide genannt.

Geeignete Sulfoniumsalze sind beispielsweise Triphenylsulfoniumsalze oder das leicht herstellbare Trimethylsulfoniumiodid. Im allgemeinen sind Phosphonium-und Ammoniumsalze den Sulfonium-und Arsoniumsalzen vorzuziehen.

Die Menge der Oniumsalze ist nicht besonders kritisch. Um eine wirksame Beschleunigung zu erzielen, reichen bereits katalytische Mengen aus, z.B. 0,5 bis 20, insbesondere 1 bis 15 Mol.%, bezogen auf den Ausgangsstoff II. Natürlich können auch größere, z.B. stöchiometrische Mengen, verwendet werden, was jedoch in der Regel keine weiteren Vorteile bringt.

Als Oxidationsmittel können die aus dem Stand der Technik bekannten Stoffe eingesetzt werden. Vorteilhaft werden Erdalkali-, insbesondere Alkalihypohalogenite wie Hypochlorite und -bromite und alkalische Hexacyanoferrat-III-Lösungen verwendet, wobei sich Natriumhypochlorite und -bromite sowie Kaliumhexacyanoferrat-III besonders bewährt haben.

Die Menge des Oxidationsmittels liegt zweckmäßigerweise bei 0,5 bis 10 mol, insbesondere 0,5 bis 1 mol, je Mol Imidazol II.

Die Oxidation der Imidazole II erfolgt in einem wäßrig-organischen Zweiphasensystem. Das Verhältnis von organischer zu wäßriger Phase ist nicht besonders kritisch, es liegt im allgemeinen bei 10:1 bis 1:1.

Als organische Phase wird ein aprotisches Lösungsmittel verwendet, z.B. Benzol, Anisol, Nitrobenzol, Benzonitril, Pyridin oder Tetrachlorkohlenstoff. Besonders bewährt haben sich nicht oder wenig polare Lösungsmittel wie Toluol, Xylol, Chloroform oder Methylenchlorid.

In den Ausgangsstoffen II

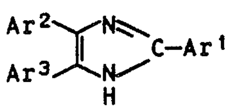

II

bedeuten die Reste $Ar^1$ bis $Ar^3$ Arylreste, beispielsweise Phenyl-oder Naphthylreste, die noch ein oder mehrere unter den Reaktionsbedingungen inerte Substituenten tragen können. Als Substituenten kommen beispielsweise Halogene, insbesondere Chlor und Brom, $C_1$-bis $C_6$-Alkyl-oder Alkoxyreste oder die Nitrogruppe in Betracht. Die Reste $Ar^2$ und $Ar^3$ können auch miteinander zu einem anellierten Ringsystem, z.B. einem Phenanthrensystem verknüpft sein.

Die Umsetzung erfolgt zweckmäßig in der Weise, daß man das Triarylimidazol II im organischen Lösungsmittel gelöst oder suspendiert vorlegt und dazu die alkalische wäßrige Phase, die das Oxidationsmittel enthält, hinzufügt. Die Oxidation wird bei guter Durchmischung der beiden Phasen vorteilhaft bei Temperaturen von 0 bis 40°C, insbesondere 0 bis 5°C durchgeführt. Sie kann kontinuierlich oder diskontinuierlich nach den dafür üblichen Techniken vorgenommen werden.

Nach dem erfindungsgemäßen Verfahren können die Triarylimidazole in kurzer Zeit auf technisch einfache und wirtschaftliche Weise zu den Hexaarylbisimidazolen oxidiert werden, die z.B. als Photoinitiatoren zur Herstellung von Filmen bzw. von Farbstoffen in transparenten Filmen verwendet werden.

Beispiele 1 bis 5

Herstellung von

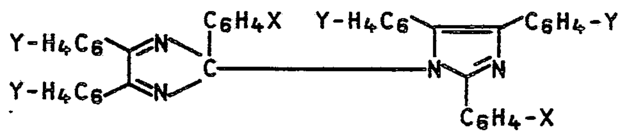

mit X = Cl und Y = p-$OCH_3$

Im organischen Lösungsmittel (LM) gelöstes 2-(2'-Chlorphenyl)-4,5-bis(4'-methoxiphenyl)imidazol wurde bei 0 bis 5°C zu einer Lösung aus Wasser, Ammoniumhalogenid und Oxidationsmittel gegeben, wobei das zweiphasige Gemisch intensiv gerührt wurde. Bei der angegebenen Temperatur wurde noch ca. 0,5 bis 2,0 h weiter gerührt (Farbumschlag von blau nach gelb) und das Reaktionsgemisch dann in an sich üblicher Weise durch Abtrennen der wäßrigen Phase, Waschen und Trocknen der organischen Phasen und Entfernen des Lösungsmittels aufgearbeitet. Anschließend wurde das Rohprodukt mit Ethanol versetzt, woraufhin es auskristallisierte oder aus Ethanol umkristallisiert (Smp. 200 bis 205°C).

Einzelheiten über die Umsetzung und Ausbeuten sind der nachstehenden Tabelle zu entnehmen. Bei Verwendung von Natriumhypochlorit als Oxidationsmittel beziehen sich die angegebenen Mengen auf eine wäßrige Hypochloritlösung ("Chlorbleichlauge") mit einem Gehalt an aktivem Chlor von 12,5 bis 13,5 %. Der pH der Lösung lag oberhalb von 12.

Tabelle

| Beispiel Nr. | Imidazol II g/mmol | Lösungsmittel ml | Oniumsalz g/mol.% a) | Oxidationsmittel | | | | Ausbeute g/% |
|---|---|---|---|---|---|---|---|---|
| | | | | $H_2O$ ml | NaOCl g | $K_3[Fe(CN)_6]$ g | KOH g | |
| 1 | 5,85/15,6 | Toluol 100 | TBAB b) 0,43/8 | 30 | 20 | - | - | 5,5/94 |
| 2 | 5,85/15,6 | Toluol 100 | TBAB 0,4/7,7 | 190 | - | 9,9 | 8,4 | 5,8/99 |
| 3 | 5,85/15,6 | Toluol 100 | BTEAC c) 0,5/14 | 30 | 20 | - | - | 5,1/88 |
| 4 | 11,6/31 | $CH_2Cl_2$ 17,5 | TBAB 0,3/3,2 | - | 19 | 1 | - | 9,5/78 |
| 5 | 55/147 | $CH_2Cl_2$ 120 | TBAB 1,5/3,3 | - | 40,7 | - | - | 49,4/90 |

a) bezogen auf II
b) Tetrabutylammoniumbromid
c) Benzyltriethylammoniumchlorid

Beispiel 6

Herstellung von

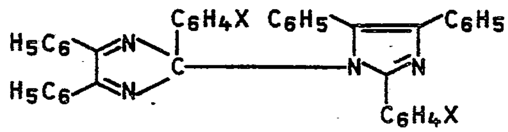

mit X = Cl

50 g (0,16 mol) 2-(2'-Chlorphenyl)-4,5-diphenylimidazol wurden in 320 ml Toluol suspendiert und bei 0 bis 25°C mit 9,6 g (0,03 mol) Tetrabutylammoniumbromid sowie 88 g Natriumhypochloritlösung (Gehalt und pH wie in den vorangehenden Beispielen) versetzt. Nach 5 Stunden wurde in üblicher Weise aufgearbeitet und das Rohprodukt aus Ether umkristallisiert. Ausbeute: 30,7 g Hexaarylbisimidazol (Smp. 210 bis 212°C) ≙ 61,4 % d.Th.

**Ansprüche**

1. Verfahren zur Herstellung von Hexaarylbisimidazolen der allgemeinen Formel I

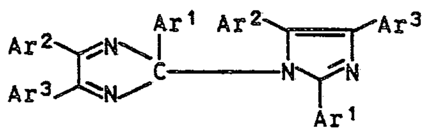

I,

in der $Ar^1$ bis $Ar^3$ Arylreste bezeichnen, wobei $Ar^2$ und $Ar^3$ auch miteinander zu einem anellierten Ringsystem verknüpft sein können, durch Oxidation der entsprechenden Triarylimidazole II

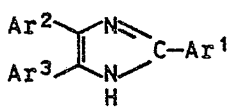

II

in einem wäßrig-organischen Zweiphasensystem,
dadurch gekennzeichnet, daß man die Oxidation in Gegenwart wirksamer Mengen eines Oniumsalzes III vornimmt.

2. Verfahren nach Anspruch 1, dadurch gekennzeichnet, daß man als Oniumsalz ein quartäres Ammonium-, Phosphonium-, Arsonium-oder ein ternäres Sulfoniumsalz verwendet.

3. Verfahren nach den Ansprüchen 1 und 2, dadurch gekennzeichnet, daß man als Oniumsalze Verbindungen der Formeln IIIa und IIIb

$R_4M^{\oplus} X^{\ominus}$ IIIa
$R_3S^{\oplus} X^{\ominus}$ IIb,

in denen die Reste R Alkyl-, Cycloalkyl-, Aryl-oder Aralkylreste bedeuten oder zwei der Alkylreste miteinander verbunden sind, M für Phosphor, Arsen oder Stickstoff steht und $X^{\ominus}$ das Anionäquivalent einer Mineralsäure darstellt, verwendet.

4. Verfahren nach den Ansprüchen 1 bis 3, daß man 0,5 bis 20 Mol.% des Oniumsalzes, bezogen auf das Triarylimidazol II, verwendet.

5. Verfahren nach den Ansprüchen 1 bis 4, dadurch gekennzeichnet, daß man als Oniumsalz ein Tetraalkyl-oder Trialkylbenzylammoniumbromid oder -chlorid verwendet.

6. Verfahren nach den Ansprüchen 1 bis 5, dadurch gekennzeichnet, daß man als Oxidationsmittel Alkali-oder Erdalkalihypohalogenite verwendet.

7. Verfahren nach den Ansprüchen 1 bis 5, dadurch gekennzeichnet, daß man als Oxidationsmittel alkalische Hexacyanoferrat-III-Lösungen verwendet.

8. Verfahren nach den Ansprüchen 1 bis 7, dadurch gekennzeichnet, daß man als organische Phase ein nicht oder wenig polares, aprotisches Lösungsmittel verwendet.

9. Verfahren nach den Ansprüchen 1 bis 8, dadurch gekennzeichnet, daß man als organische Phase halogenierte Kohlenwasserstoffe oder aromatische Kohlenwasserstoffe verwendet.